# EUROPEAN PATENT APPLICATION

(11) **EP 4 257 673 A1**
(43) Date of publication of application: **11.10.2023**
(21) Application number: 21901105.3
(22) Date of filing: 06.12.2021
(51) Int. Cl.: C12N 5/0775, A61K 8/98, A61Q 19/00, A61K 35/51, A61K 9/00, A61P 17/02, A61P 29/00

(54) **METHOD FOR PREPARING HIGH-CONCENTRATION STEM CELL EXOSOMES WITH ENHANCED ANTI-INFLAMMATORY AND REGENERATIVE FUNCTIONS USING LIPOPOLYSACCHARIDE AND LIPOTEICHOIC ACID**

(30) Priority: 04.12.2020 KR 20200168689
(71) Applicant: Primoris Co., Ltd., Gyeonggi-do 14322 (KR)
(72) Inventor: LEE, Jae-yong, Gwangju-si Gyeonggi-do 12777 (KR); AHN, Hee-jin, Seoul 02598 (KR); LEE, Yea-in, Gwangmyeong-si Gyeonggi-do 14247 (KR); LEE, Dong-yeol, Seoul 06732 (KR); NA, Kyu-heum, Suwon-si Gyeonggi-do 16707 (KR)
(74) Representative: ZBM Patents - Zea, Barlocci & Markvardsen
(86) International application number: PCT/KR2021/018354
(87) International publication number: WO 2022/119417

(57) **Abstract**

The present invention relates to a method for producing high-concentration stem cell exosomes with enhanced anti-inflammatory function using Lipopolysaccharide (LPS) and/or Lipoteichoic Acid (LTA), as well as anti-inflammatory and/or regenerative compositions that can be produced by the aforementioned method.

The production method for the anti-inflammatory composition according to the present invention includes a first step of treating stem cells during cultivation with one or more selected from a group consisting of Lipopolysaccharide (LPS) and Lipoteichoic Acid (LTA); and optionally, a second step of separating stem cells, their culture medium, and/or their exosomes obtained as a result of the first step.

## Description

### Field of the Invention

The present invention relates to a method for producing high-concentration stem cell exosomes with enhanced anti-inflammatory and/or regenerative functions using LPS (Lipopolysaccharide) and/or LTA(LipoteichoicAcid). It also relates to an anti-inflammatory and regenerative-inducing composition producible by the aforementioned method.

### Background of the Invention

The cells that make up our body secrete various sizes of extracellular vesicles (EVs) with lipid bilayers as signaling molecules for communication with neighboring cells. EVs are classified into different categories based on their size: exosomes (30-200nm), microvesicles (200-1000nm), and apoptotic bodies (1µm-5µm).

Exosomes contain nucleic acids such as miRNA and proteins. Research on exosomes secreted by adult stem cells has shown their superior efficacy in promoting tissue regeneration and anti-inflammatory effects on neighboring cells. Consequently, studies are being conducted to develop various therapeutic agents utilizing these properties.

Methods to enhance the functionality of exosomes include modulating the characteristics of the exosome-releasing cells and loading specific substances into isolated exosomes for their use as transporters of therapeutic agents.

Various techniques are available to isolate exosomes, including ultracentrifugation, ultrafiltration, and chromatography. The choice of isolation method can influence the functional properties, purity, concentration, and size of the exosomes.

There are different types of stem cells, including embryonic stem cells, adult stem cells, and induced pluripotent stem cells. Among them, adult stem cells, which can be extracted from sources such as blood, bone marrow, and adipose tissue, are ethically unrestricted compared to embryonic stem cells. Stem cells derived from bone marrow and adipose tissue are obtained invasively from donors, while umbilical cord blood stem cells are extracted from discarded umbilical cords after birth, without invasive procedures. Additionally, umbilical cord blood stem cells have the advantage of minimal efficacy differences among donors. Research is being conducted on developing techniques to enhance the skin penetration of effective factors in stem cells, and one of these techniques involves the use of exosomes.

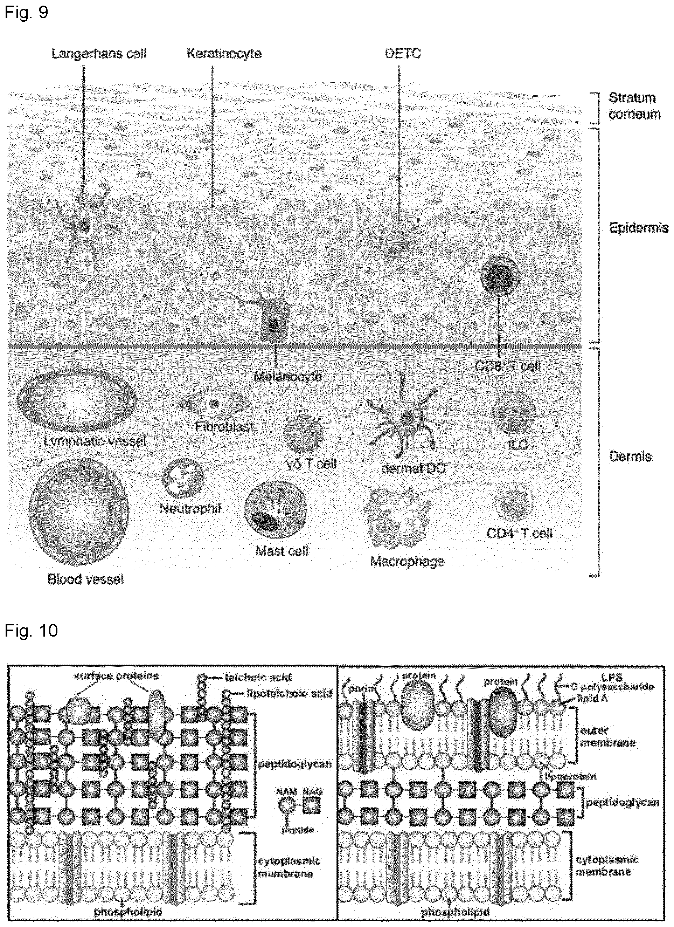

The skin is composed of two layers, the epidermis and the dermis(Fig. 9).

The epidermis is filled with various types of cells, including keratinocytes, melanocytes, dendritic cells, and tactile cells. The majority of epidermal cells are keratinocytes, which play a crucial role in forming a barrier against environmental damage caused by heat, UV radiation, moisture loss, pathogenic bacteria, fungi, parasites, and viruses. Several structural proteins (filaggrin, keratin), enzymes (proteases), lipids, and antimicrobial peptides (defensins) contribute to maintaining the important barrier function of the skin.

The dermis, which is 15 to 40 times thicker than the epidermis (ranging from 0.04mm to 1.6mm), occupies most of the skin and acts as the connective tissue between the epidermis and subcutaneous tissue. It contains skin appendages, blood vessels, lymphatic vessels, muscles, nerves, and is divided into two layers: the papillary dermis and the reticular dermis.

The papillary dermis occupies only a small portion of the overall dermal thickness and is composed of collagen, blood vessels, and fibrocytes. It supplies nutrients to the avascular basal layer of the epidermis. The reticular dermis, which comprises the majority of the dermis, provides strength, flexibility, and elasticity to the skin. It consists of densely arranged elastic and collagen fibers, as well as various matrix proteins. Collagen, which accounts for approximately 25% of the total protein in connective tissue, plays a significant role in the tensile strength of the dermis. On the other hand, matrix metalloproteinases (MMPs) are zinc-dependent endopeptidases that hydrolyze the extracellular matrix (ECM). MMPs promote the circulation of skin tissue by degrading the ECM, but they also contribute to wrinkle formation, skin aging, and the promotion of cancer cell metastasis. Their formation is stimulated by factors such as UV radiation, stress, and antibiotics. Fibronectin is a glycoprotein that exists on cell surfaces, in connective tissue, and in blood. Cells are indirectly connected to collagen through fibronectin.

Fibroblasts produce and secrete growth factors and cytokines required by keratinocytes. Keratinocytes are the most exposed cells in the environment as epidermal cells. Fibroblasts can be activated through keratinocytes and, in turn, remodel the extracellular matrix to maintain the evenness of the skin layer.

The skin is the largest organ that covers the entire body. Keratinocytes (KCs) are the major components of the skin and play a crucial role in producing keratin proteins, which form a protective barrier against external stimuli. It has been demonstrated that KCs produce various types of cytokines, indicating the important role of the skin in the body's immune and inflammatory responses. Cytokines mediate cell growth, differentiation, inflammation, and immune reactions, exerting influences on other cells and organs. Therefore, cytokines contribute to maintaining cellular and intercellular homeostasis. Dysregulation and abnormal production of cytokines have been detected in various skin disorders. Accumulating evidence shows the significant contribution of cytokines to the etiology or severity of specific diseases.

The human skin is located on the outermost part of the body and serves as a barrier to protect the body from external stressors. However, it is also an organ that generates reactive oxygen species (ROS) in response to external stimuli such as environmental pollution and ultraviolet radiation.

Ultraviolet (UV) radiation, which can easily penetrate the skin, has beneficial effects such as maintaining cellular density, vitamin D synthesis, and antimicrobial actions. However, when reaching the dermis, it increases reactive oxygen species (ROS) within dermal fibroblasts, inducing the expression of matrix metalloproteinases (MMPs). This leads to the degradation of collagen, elastin, glycosaminoglycan (GAG), and hyaluronic acid chains in connective tissue, ultimately resulting in the formation of skin wrinkles and accelerating skin aging. Furthermore, lipid peroxidation caused by ROS can extend beyond the site of occurrence and peroxide radicals induce peroxidation in distant areas through the bloodstream. These oxidative reactions contribute to hypertension, atherosclerosis, heart failure, rheumatoid arthritis, allergies, cancer, stroke, brain disorders such as Parkinson's disease and dementia, and osteoporosis. Additionally, ROS is involved in the development or exacerbation of atopic dermatitis, acne, psoriasis, as well as inflammatory processes and allergic responses, causing DNA damage within cells and promoting skin cancer and skin aging.

Nitric oxide (NO) and prostaglandin E2 (PGE2) are representative inflammatory mediators in immune cells. NO serves as a signaling molecule involved in maintaining cellular homeostasis, neurotransmitter transport, anti-cancer effects, and cytotoxicity. However, excessive presence of NO can lead to cellular damage and inflammatory responses. PGs, including PGE2, play a role in maintaining homeostasis, vasodilation, smooth muscle stimulation, and inflammation mediation. PGE2, like NO, has dual effects by protecting cells from apoptosis while inducing inflammation and pain.

Bacteria are the most abundant microorganisms thriving in various life forms. Found in humans, animals, and soil, bacteria are single-celled organisms that can divide every 20 minutes and survive under suitable conditions. Certain bacteria in the body produce vitamin K and assist in food digestion within the intestines. Other bacteria are used in the production of fermented foods like yogurt and kimchi. Some bacteria play a crucial role in purifying contaminated environments and controlling harmful bacteria, making them essential and indispensable to us. However, a small fraction of pathogenic bacteria can cause various bacterial diseases such as pneumonia and even lead to severe complications like sepsis, resulting in the death of the host. Sepsis induces damage to cells, organs, and tissues by triggering a cytokine storm.

Bacteria retain characteristics of prokaryotes and lack structures such as nuclear membranes, mitochondria, and chloroplasts. From an immunological perspective, the most notable aspect of bacterial structure is the cell wall, which maintains size, shape, and prevents cell lysis caused by osmotic pressure.

The cell wall of Gram-negative bacteria is composed of lipopolysaccharide (LPS) and peptidoglycan(Fig. 10). It induces strong immune activity even in minute amounts. Lipid A and various oligosaccharides make up the LPS structure. On the other hand, Gram-positive bacteria have a cell wall component called lipoteichoic acid (LTA), which has a similar structure to LPS in Gram-negative bacteria. Recent studies have revealed that the LTA of probiotic bacteria can modify the production of intestinal bacteria and mucin, leading to the alleviation of intestinal inflammation. Many studies are currently underway to explore substances that promote the secretion of anti-inflammatory molecules in cells and their association with anti-inflammatory efficacy.

### BRIEF SUMMARY OF THE INVENTION

The present invention aims to provide high-concentration exosomes with enhanced anti-inflammatory and regenerative functions by inducing a mild inflammatory response in stem cells during exosome production through the pre-treatment of lipopolysaccharide (LPS) and lipoteichoic acid (LTA). This process activates the immune response of stem cells, followed by the secretion of exosomes that induce anti-inflammatory responses.

A first aspect of the present invention provides a method of producing exosomes comprising:
(a) a step of culturing umbilical cord blood stem cells in a serum-free medium supplemented with one or more selected from a group consisting of lipopolysaccharide (LPS) and lipoteichoic acid (LTA) to increase the concentration of exosomes secreted during the culture of umbilical cord blood stem cells compared to untreated umbilical cord blood stem cells; and
(b) optionally, a step of separating untreated umbilical cord blood stem cells, their culture conditioned medium, and/or their exosomes obtained from step (a).

The exosomes may have enhanced anti-inflammatory and/or regenerative functions compared to exosomes derived from untreated umbilical cord blood stem cells.

A second aspect of the present invention provides a composition for topical application to the skin or other covering and lining membranes of the body, which contains exosomes obtained by the production method of the first aspect of the present invention, or a culture medium containing such exosomes as active ingredients.

The composition for topical application to the skin or other covering and lining membranes can be a cosmetic composition, a non-prescription pharmaceutical composition, or a pharmaceutical composition.

In the composition for topical application to the skin or other covering and lining membranes, exosomes can be used as a transdermal carrier.

A third aspect of the present invention provides a method of producing an anti-inflammatory and/or regenerative composition comprises the following steps: (i) treating stem cells during cultivation with one or more selected from the group consisting of lipopolysaccharide (LPS) and lipoteichoic acid (LTA); and (ii) optionally, separating stem cells, their culture medium, and/or their exosomes obtained from step (i).

In step (i), the production of exosomes secreted by stem cells can be increased by lipopolysaccharide (LPS) and/or lipoteichoic acid (LTA).

A fourth aspect of the present invention provides an anti-inflammatory and/or regenerative composition obtained by production method according to the third aspect of the present invention.

### BRIEF DESCRIPTION OF THE DRAWINGS/FIGURES

The patent or application file contains at least one drawing executed in color. Copies of this patent or patent application publication with color drawing(s) will be provided by the Office upon request and payment of the necessary fee.
Fig. 1 represents a graph depicting the expression levels of anti-inflammatory cytokines in umbilical cord blood stem cells pre-treated with LPS and LTA and culture conditioned medium thereof.
Fig. 2 illustrates a graph showing the concentration and size of exosomes produced from umbilical cord blood stem cells pre-treated with LPS and LTA.
Fig. 3 displays a graph analyzing the levels of inflammatory-related factors contained within exosomes produced from stem cells pre-treated with LPS and LTA.
Fig. 4 depicts a graph analyzing cell viability through Cell Viability analysis after exosome treatment on human keratinocytes induced with inflammation.
Fig. 5 presents a graph analyzing the expression of pro-inflammatory cytokines in human keratinocytes induced with inflammation after exosome treatment.
Fig. 6 showcases a graph analyzing the expression of anti-inflammatory cytokines in human keratinocytes induced with inflammation after exosome treatment.
Fig. 7 represents a graph analyzing the rate of cell proliferation in skin cells and keratinocytes treated with exosomes.
Fig. 8 shows images obtained by staining and capturing cells to confirm the promotion of cell mobility in skin cells and keratinocytes treated with exosomes.
Fig. 9 provides a schematic representation of various cell types residing in the skin.
Fig. 10 presents a conceptual diagram illustrating the cell wall structure of gram-positive bacteria (left) and gram-negative bacteria (right).

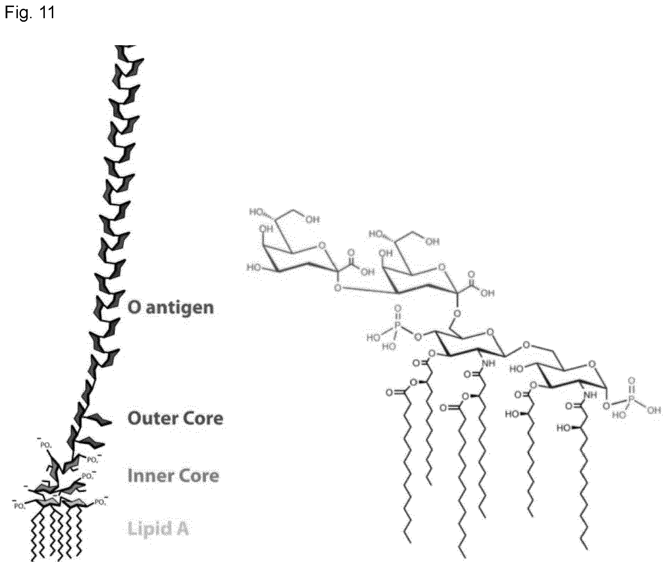

Fig. 11 displays the structure of lipopolysaccharide (LPS), with Kdo residues in red (core), glucosamine residues in blue, acyl chains in black, and phosphate groups in green.

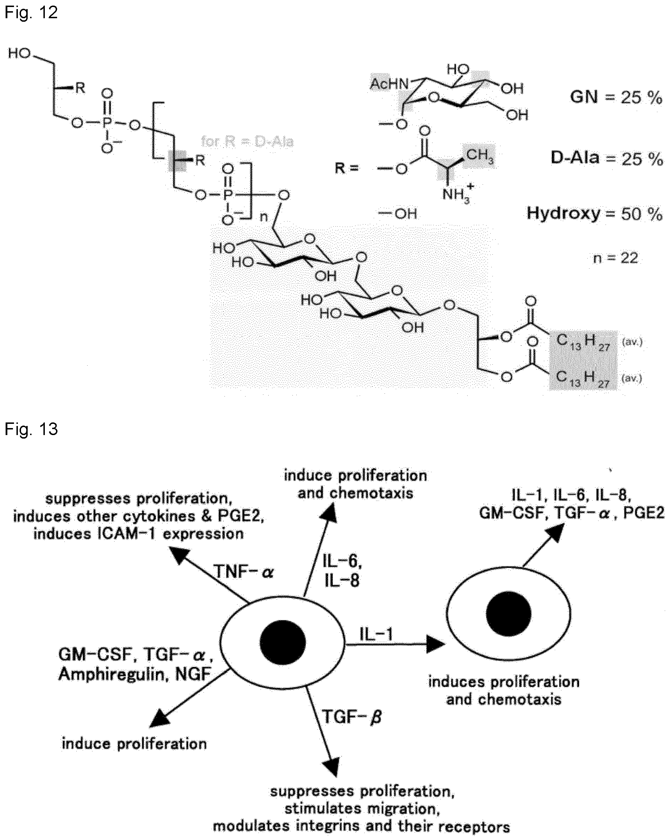

Fig. 12 represents the structure of lipoteichoic acid (LTA) from S. aureus.
Fig. 13 depicts the diverse effects of cytokines derived from KC (Keratinocyte)-source.

### DETAILED DESCRIPTION OF THE INVENTION

The method for producing exosomes according to the present invention comprises:
(a) a step of culturing umbilical cord blood stem cells in a serum-free medium supplemented with one or more selected from a group consisting of lipopolysaccharide (LPS) and lipoteichoic acid (LTA) to increase the concentration of exosomes secreted during the culture of umbilical cord blood stem cells compared to untreated umbilical cord blood stem cells; and
(b) optionally, a step of separating untreated umbilical cord blood stem cells, their culture conditioned medium, and/or their exosomes obtained from step (a).

The method of producing an anti-inflammatory and/or regenerative composition according to the present invention comprises:
(i) treating stem cells during cultivation with one or more selected from the group consisting of lipopolysaccharide (LPS) and lipoteichoic acid (LTA); and
(ii) optionally, separating stem cells, their culture medium, and/or their exosomes obtained from step (i).

According to the present invention, exosomes released from umbilical cord blood stem cells pre-treated with LPS and LTA demonstrated superior anti-inflammatory and regenerative efficacy compared to exosomes derived from normally cultured stem cells. The concentration of exosomes was also found to be higher, while their size was comparable to the control group. Furthermore, when these exosomes with enhanced anti-inflammatory and regenerative functions according to the present invention were applied to cells, no cytotoxicity or induction of inflammatory responses was observed. Additionally, the treatment with these exosomes resulted in increased proliferation and cellular mobility of skin cells and keratinocytes, indicating their potential for tissue regeneration. Therefore, the produced, isolated, and purified exosomes according to the present invention can be effectively utilized as anti-inflammatory and regenerative pharmaceutical compositions or as compositions for topical application on the skin or other covering and lining membranes. The present invention has been completed based on these findings.

In summary, the present invention relates to the production of high-concentration stem cell exosomes with enhanced anti-inflammatory and/or regenerative functions. This is achieved by inducing the inflammatory response of stem cells through pre-treatment with lipopolysaccharide (LPS) and/or lipoteichoic acid (LTA) during cultivation, leading to activation of the immune response of stem cells, specifically inducing inflammation, and subsequently inducing an anti-inflammatory response.

Typically, inflammatory responses serve as a defense mechanism to protect the body against microbial infection or external damage and are associated with innate immunity and disease.

In this specification, stem cells induced with anti-inflammatory responses by treatment with lipopolysaccharide (LPS) and/or lipoteichoic acid (LTA) during stem cell cultivation may exhibit increased mRNA expression and/or secretion of at least one anti-inflammatory cytokine compared to untreated stem cells. Additionally, the exosomes secreted by these stem cells may contain higher levels of at least one anti-inflammatory cytokine compared to untreated stem cells.

The exosomes of the present invention can be secreted from stem cells with induced anti-inflammatory response by pre-treating with lipopolysaccharide (LPS) and/or lipoteichoic acid (LTA) during stem cell culture to induce a mild inflammatory response in the stem cells and activate their immune response. Alternatively, the exosomes of the present invention may be obtained by subjecting the stem cells with induced anti-inflammatory response to physical forces, such as crushing or fragmentation.

Moreover, by inducing exosome secretion from umbilical cord blood stem cells, which are already known to be rich in substances involved in tissue regeneration, it is possible to obtain exosomes with high concentrations that naturally incorporate relevant substances. These exosomes exhibit both anti-inflammatory and tissue regeneration functions.

Within this specification, extracellular vesicles that exhibit enhanced anti-inflammatory and/or regenerative functions also fall within the scope of the exosomes of the present invention.

Within this specification, there is a broader concept of the skin known as the covering and lining membranes of the body. The covering and lining membranes consist of continuous multicellular sheets of epithelial tissue that are attached to the underlying layer of connective tissue. These covering and lining membranes include the cutaneous membrane (skin), mucous membranes, and serous membranes.

### 1. Lipopolysaccharide (LPS) and Lipoteichoic Acid (LTA)

Within this specification, Lipopolysaccharide (LPS) can be a component of the cell wall of Gram-negative bacteria, while lipoteichoic acid (LTA) can be a component of the cell wall of Gram-positive bacteria.

Bacteria can be classified into two major groups based on the composition of their cell wall(Fig. 10). One group consists of Gram-negative bacteria that have lipopolysaccharide (LPS) on the outer layer and peptidoglycan between the cell wall and cell membrane. The other group includes Gram-positive bacteria that have peptidoglycan and lipoteichoic acid (LTA). In Gram-positive bacteria, the cell wall is thickly covered with multiple layers of peptidoglycan, which accounts for approximately 80-90% of the cell wall. On the other hand, Gram-negative bacteria have a single thin layer of peptidoglycan, which constitutes only 10-20% of the cell wall. The outer cell wall of Gram-negative bacteria is composed of a form surrounded by an outer membrane composed of phospholipids, lipopolysaccharide, lipoprotein, and the like.

Examples of Gram-positive bacteria include Staphylococcus aureus, Streptococcus pyogenes, Streptococcus pneumoniae, Mycobacterium leprae, Corynebacterium diphtheriae, Clostridium tetani, Clostridium botulinum, and Bacillus anthracis. These bacteria are generally more susceptible to dyes and antibiotics, and they require amino acids and vitamins for their metabolism. Some of them release exotoxins, which are highly potent in specific bacterial strains but easily destroyed by heat. These toxins exhibit high antigenicity within the body, and when specific antibodies bind to them, they can neutralize or eliminate the toxic function of the exotoxins. Furthermore, by treating the exotoxins with specific processes (e.g., formalin treatment), their toxicity can be eliminated, allowing for the production of a large amount of toxin-neutralizing antibodies.

Probiotics, which possess a cell wall structure similar to pathogenic bacteria but do not induce septicemia, exhibit functions that inhibit the intraorganismal infection and proliferation of harmful bacteria.

Gram-negative bacteria are generally resistant to dyes such as triphenylmethane and acriflavine, as well as to surfactants. They have simple nutritional requirements for survival and can grow well in simple culture media. Their toxins, known as endotoxins, are not easily destroyed by heat. These toxins can induce antibody responses and have been found to exhibit a wide range of immunogenicity. Lipopolysaccharide (LPS), an endotoxin that forms the membrane structure of Gram-negative bacteria, plays a crucial role in the immune response of macrophages. The activation of macrophages by LPS induces the production of various inflammatory mediators, leading to the generation of arachidonic acid, prostaglandins (PGs), and nitric oxide (NO), making it widely used in the study of inflammatory responses. Substances that directly inhibit the activity of inflammatory mediators such as NO and PGE2 have high potential for the development of anti-inflammatory agents that can suppress various inflammation-related conditions, including acne, atopic dermatitis, rheumatoid arthritis, and malignant neoplasms.

The cell wall components of bacteria play a crucial role in immune response research as they are the first recognized targets by the human or animal body. Lipopolysaccharide (LPS), a representative immune-active factor of gram-negative bacteria, is known to play a vital role in the interaction between pathogenic bacteria and eukaryotes. The mechanisms underlying sepsis induced by LPS are well understood in animal models and humans. LPS, composed of lipid A and various oligosaccharides, can induce potent immune activity even in trace amounts. After binding to LPS-binding protein (LBP) and CD14 receptor in the body, it activates toll-like receptor (TLR) 4, leading to the production of interferon (IFN)-β. IFN-β binds to cell receptors, phosphorylates STAT1, induces the production of inducible nitric oxide synthase (iNOS), and ultimately generates nitric oxide (NO) by activating myosin phosphatase and potassium channels in vascular cells, resulting in vasodilation. NO is closely associated with septic shock.

Gram-positive bacteria lack LPS, but despite being a major cause of sepsis, the underlying mechanisms have not been fully elucidated. However, a gram-positive bacterial cell wall component, known as lipoteichoic acid (LTA), has long been recognized as an inducer of septic shock. Lipoteichoic acid (LTA) consists of a lipid portion composed of fatty acids, similar to LPS, and it also contains various carbohydrates.

The structure of LTA can vary depending on the species of gram-positive bacteria and may include a long chain of ribitol or glycerolphosphate. LTA is anchored to the cell membrane via diacylglycerol. It serves as a regulator of autolytic wall enzymes called muramidases. LTA possesses antigenic properties that can stimulate specific immune responses.

LTA can bind to target cells non-specifically through membrane lipids or specifically to CD14 and Toll-like receptors. Binding to TLR-2 has been shown to induce the expression of NF-κB (central transcription factor), leading to increased expression of pro- and anti-apoptotic genes. Its activation also induces the activation of mitogen-activated protein kinases (MAPK) along with the activation of phosphoinositide 3-kinase.

The molecular structure of LTA has been found to have the strongest hydrophobic binding among all bacterial components. LTA can upregulate the levels of PD-1 in monocytes, leading to the induction of IL-10 production by monocytes upon binding of PD-1 by PD-L. This, in turn, triggers IL-10-dependent suppression of CD4 T-cell expansion and function.

Table 1 compares the chain length and substitutions of commercially available or experimentally extracted (using butanol) LTA from S. aureus and B. subtilis.

**[Table 1]**

| LTA type | GN (%) | D-Ala (%) | D-Ala/GN ratio | *n* |
|---|---|---|---|---|
| *S*. *aureus* (Sigma) | 5 | 20 | 2.5 | 25 |
| *S*. *aureus* (butanal-extracted) | 15 | 70 | 4.8 | 48 |
| *B. subtilis* (Sigma) | 20 | 5 | 0.4 | ND |
| *B. subtilis* (butanol-extracted) | 25 | 25 | 1.0 | 22 |

| | | | | |
|---|---|---|---|---|
| *^{a}* Degrees of substitutions (*n*, chain length of LTA-glycerophosphate) were determined from the ¹H NMR integrals of LTA. The high degree of nucleic acid contamination prevented chain length determination (ND, not determined) by NMR in case of the commercial *B. subtilis* LTA. | | | | |

The immune response to microbial infections can be categorized into innate immunity and acquired immunity. Acquired immunity exhibits high specificity towards antigens and is capable of efficiently eliminating invading microorganisms. However, it requires time to function optimally. In contrast, innate immunity recognizes a set of patterned antigens known as pathogen-associated molecular patterns (PAMPs) during the early stages of microbial infection. It plays a role in the removal of invading microorganisms and efficiently triggers acquired immunity. Polysaccharide antigens, LTA, and LPS serve as common antigens corresponding to PAMPs, which induce immune responses through toll-like receptors (TLRs). Specifically, LTA and LPS bind to binding proteins or carrier proteins, activate TLRs, and induce the expression of inflammatory cytokines such as TNF-α. Moreover, in severe bacterial infections, excessive secretion of highly toxic inflammatory factors occurs due to hypersensitive reactions of immune cells. In this context, LPS and LTA play a crucial role in sepsis syndrome, which can lead to fatal damage to multiple organs and the death of patients.

Another distinctive feature of the present invention is that the high-concentration stem cell-derived exosomes with enhanced anti-inflammatory functions, which are produced by treating stem cells during cultivation with lipopolysaccharide (LPS) and/or lipoteichoic acid (LTA), do not contain LPS and/or LTA.

In this specification, "the exosomes do not contain LPS and/or LTA" means that cell viability is not decreased when the cells are treated with the exosomes.

According to the present invention, high-concentration stem cell-derived exosomes produced by treating stem cells with lipopolysaccharide (LPS) and/or lipoteichoic acid (LTA) during cultivation, reduces the expression of pro-inflammatory factors and/or increases the expression of anti-inflammatory factors (e.g., TGF-β1 and IL-10) when applied to cells.

Furthermore, the exosomes secreted contain regenerative factors unique to umbilical cord blood stem cells, along with anti-inflammatory factors. Therefore, when these exosomes are applied to skin cells or keratinocytes, they have the ability to increase cell proliferation and mobility.

### 2. Proinflammatory Cytokines as Inflammatory Inducing Factors

Proinflammatory cytokines, also known as inflammatory cytokines, are immune-regulatory cytokines that favor inflammatory responses. They play a role in initiating inflammation and mediating innate immune responses, regulating host defense against pathogens.

Proinflammatory cytokines are primarily generated and involved in the upregulation of inflammatory responses. They are a type of signaling molecules (cytokines) secreted by immune cells such as helper T cells (Th) and macrophages, as well as other specific cell types that promote inflammation. Examples of proinflammatory cytokines include interleukin-1 (IL-1), IL-12, IL-18, tumor necrosis factor-alpha (TNF-α), interferon-gamma (IFNγ), and granulocyte-macrophage colony-stimulating factor (GM-CSF).

Interleukin-1 (IL-1) and tumor necrosis factor (TNF) are proinflammatory cytokines that, when administered to humans, can induce fever, inflammation, tissue destruction, and in some cases, shock and death. IL-1 and TNF are inducers of endothelial adhesion molecules, which are essential for leukocytes to adhere to the endothelial surface and migrate into tissues. Proinflammatory cytokines such as IL-1β, IL-6, and TNF-α also contribute to pathological pain. IL-1β is released by monocytes and macrophages but is also present in nociceptive dorsal root ganglion (DRG) neurons. IL-6 plays a role in neuronal reactions to injury. TNF-α is a well-known proinflammatory cytokine present in neurons and glial cells. TNF-α often interacts with other signaling pathways to regulate apoptosis in cells.

Due to their proinflammatory actions, proinflammatory cytokines can induce fever, inflammation, tissue destruction, and in some cases, shock and even death, exacerbating the disease itself or disease-related symptoms. Excessive amounts of proinflammatory cytokines have detrimental effects.

Moreover, excessive chronic production of inflammatory cytokines contributes to inflammatory diseases, which are associated with other conditions such as atherosclerosis and cancer. Dysregulation is also associated with depression and other neurological disorders. Maintaining a balance between proinflammatory and anti-inflammatory cytokines is crucial for maintaining health. Aging and exercise also influence the levels of inflammation through the release of proinflammatory cytokines.

Therapeutic approaches for treating inflammatory diseases include the use of monoclonal antibodies that neutralize proinflammatory cytokines or their receptors.

### 3. Anti-inflammatory Cytokines and Cytokine Inhibitors as Inflammation Inhibitors

The overall effect of an inflammatory response is determined by the balance between proinflammatory cytokines and anti-inflammatory cytokines.

Inflammatory/immune responses have both protective and damaging aspects, so they need to be precisely regulated quantitatively, temporally, and selectively in addition to tissue or stimulus-specific control. Once the issue is resolved, the response should be terminated. Therefore, the action of anti-inflammatory drugs should also be precisely controlled in terms of quantity, quality, and timing to aid in the treatment of diseases. If the drugs fail to harmoniously and accurately regulate the inflammatory response, they may exacerbate damage, worsen the disease, or even induce new diseases. Thus, an accurate understanding of the inflammatory response is essential as a prerequisite for the treatment and prevention of diseases through the regulation of inflammatory responses.

Key anti-inflammatory cytokines and cytokine inhibitors are listed in Table 2. Anti-inflammatory cytokines are cytokines that possess the ability to inhibit the synthesis of IL-1, tumor necrosis factor (TNF), and other major proinflammatory cytokines.

**[Table 2]**

| **Cytokines** | | **Major Activities** |
|---|---|---|
| II-1ra | Specific inhibitor of IL-1α and IL-1β mediated cellular activation at the IL-1 cellular receptor level | |
| IL-4 | Promotes Th2 lymphocyte development; inhibition of LPS-induced proinflammatory cytokines synthesis | |
| IL-6 | Inhibition of TNF and IL-1 production by macrophages | |
| IL-10 | Inhibition of monocyte/macrophage and mrutrophil cytokine production and inhibition of TH1-type lymphocyte responses | |
| IL-11 | Inhibits proinflammatory cytokines response by monocyte/macrophages and promotes Th2 lymphocyte response | |
| IL-13 | Shares homology with IL-4 and shares IL-4 receptor; attenuation of monocyte/macrophage function | |
| TGF-β | Inhibition of monocyte/macrophage MHC, class II expression and proinflammatory cytokines synthesis | |

### 4. Stem Cell Culture Medium

In this specification, the term "culture conditioned medium" can refer to the cell culture supernatant, or equivalent obtained after culturing stem cells. The stem cell culture medium contains various bioactive substances, including exosomes secreted from cells, during the stem cell culture process. Furthermore, the aforementioned bioactive substances collectively refer to cytokines, growth factors, immune modulators, or other factors that can affect the functions of cells or the body.

Another feature of the present invention is that when lipopolysaccharide (LPS) and/or lipoteichoic acid (LTA) are treated during stem cell culture, the production of exosomes secreted by stem cells increases in the culture medium. These exosomes, derived from stem cells treated with lipopolysaccharide (LPS) and/or lipoteichoic acid (LTA) exhibit enhanced anti-inflammatory properties compared to exosomes derived from untreated stem cells. Therefore, stem cells treated with lipopolysaccharide (LPS) and/or lipoteichoic acid (LTA), their culture medium, and/or their exosomes can be used as effective components in compositions for topical application to the skin or other covering and lining membranes of a living body, as well as in compositions for anti-inflammatory purposes.

Furthermore, as mentioned earlier, inflammation/immune response has both protective and detrimental aspects, and the overall effect of an inflammatory response is determined by the balance between pro-inflammatory cytokines and anti-inflammatory cytokines. Therefore, the action of anti-inflammatory drugs should be precisely controlled in terms of quantity, quality, and timing. Accordingly, by culturing stem cells with lipopolysaccharide (LPS) and/or lipoteichoic acid (LTA) to mass-produce exosomes with enhanced anti-inflammatory functions in vitro, it is possible to administer them in vivo by adjusting the desired timing, application site, and/or dosage.

Adult stem cells are undifferentiated cells that have the ability to differentiate into specific cell types of a particular tissue when needed. Adult stem cells can include mesenchymal stem cells, mesenchymal stromal cells, or multipotent stem cells, among others, and are not limited to these types. One advantage of adult stem cells is that they can be extracted from already developed tissues in the body, such as bone marrow or brain cells, avoiding ethical debates unlike embryonic stem cells derived from human embryos. In this invention, adult stem cells can be derived from sources such as umbilical cord, umbilical cord blood, bone marrow, adipose tissue, muscle, nerve, skin, membranes, or placenta, but are not limited to these sources.

Mesenchymal stem cells secrete various growth factors and cytokines, such as epithelial growth factor (EGF) and fibroblast growth factor (FGF).

Stem cells derived from umbilical cord blood have the advantage of using cord blood formed during the donor's gestational period (40 weeks), which eliminates the variability in efficacy based on the donor's condition, unlike adipose tissue or bone marrow-derived adult stem cells.

A culture medium refers to a composition that contains essential components necessary for the growth and proliferation of cells in vitro. It encompasses stem cell culture media commonly used in the field, such as DMEM (Dulbecco's Modified Eagle's Medium), MEM (Minimal Essential Medium), BME (Basal Medium Eagle), RPMI 1640, DMEM/F-10 (Dulbecco's Modified Eagle's Medium: Nutrient Mixture F-10), DMEM/F-12 (Dulbecco's Modified Eagle's Medium: Nutrient Mixture F-12), α-MEM (α-Minimal essential Medium), G-MEM (Glasgow's Minimal Essential Medium), IMDM (Iscove's Modified Dulbecco's Medium), KnockOut DMEM, as well as commercially manufactured media or artificially synthesized media. It is not limited to these examples. In this specification, a culture medium generally includes carbon sources, nitrogen sources, trace elements, amino acids, antibiotics, and various growth factors, among other components.

### 5. Stem Cell-Derived Exosomes

The composition for topical application on the skin or other covering and lining membranes, as well as the anti-inflammatory and/or regenerative composition of the present invention, can include stem cell-derived exosomes resulting from the treatment of stem cells with lipopolysaccharide (LPS) and/or lipoteichoic acid (LTA). These exosomes can be in the form of exosomes released by stem cells or exosome-containing culture conditioned medium.

Exosomes are considered ideal nanovesicles for drug delivery due to their ability to cross cell membranes and transport substances. The lipid bilayer vesicle system of exosomes has been evaluated as one of the most effective strategies for delivering drugs to the dermal layers of the skin, overcoming skin penetration issues (Saahil Arora et al., Asian Journal of Pharmaceutics, 6, 4, 237-244, 2012).

Furthermore, exosomes contain RNA, proteins, lipids, and metabolites that reflect the cell type of their origin. Exosomes contain various molecular components (e.g., proteins and RNA) specific to the cell and tissue of their origin. Although the protein composition of exosomes varies depending on the cell and tissue, most exosomes contain a conserved set of proteins.

Exosomes can be isolated from the culture medium obtained after culturing cells, and their size and content can be influenced by molecular signals received by the cells producing the exosomes. According to the present invention, exosomes obtained after culturing pre-treated umbilical cord stem cells with LPS and/or LTA exhibit superior anti-inflammatory and/or regenerative efficacy compared to exosomes isolated from conventionally cultured stem cells. Additionally, the concentration of exosomes is higher. Furthermore, the size of the exosomes is not significantly different from the untreated control group.

The present invention can utilize the culture medium itself as a raw material without isolating exosomes, but it is also possible to isolate exosomes for use. Additionally, for quality assurance and other purposes, exosomes can be separated to confirm their size properties within the culture medium.

Methods such as centrifugation, immunoprecipitation, and filtration can be utilized to isolate exosomes from the culture medium.

Among the exosome isolation methods, the most commonly used method, ultracentrifugation, cannot separate a large quantity of exosomes at once, requires expensive equipment, takes a considerable amount of time for isolation, and can physically damage exosomes due to strong centrifugal forces. Moreover, it has drawbacks such as lower purity of isolated exosomes. To address these limitations, the PS (phosphatidylserine) affinity method has been developed, which utilizes substances that specifically bind to phosphatidylserine, a protein present in the membrane of exosomes, to increase the purity of isolated exosomes. However, this method has the disadvantage of lower yield compared to ultracentrifugation. Another method, filtration, involves passing the culture medium through pores of a size that selectively separates and concentrates only the exosomes. The tangential flow filter (TFF) is a representative technique for filtration and is currently known as the safest method for exosome isolation.

Exosomes in the culture conditioned medium of umbilical cord blood-derived mesenchymal stem cells (UCB-MSC) contains a higher level of various growth factors such as EGF, VEGF, TGF, HGF, FGF, IGF, and PDGF compared to exosomes from adipose tissue or bone marrow-derived mesenchymal stem cells. Growth factors like EGF promote the proliferation of fibroblasts, which are the skin's constituent cells, and enhance cell migration and collagen synthesis. Therefore, UCB-MSC-derived exosomes can exhibit excellent effects in improving skin conditions such as skin regeneration, enhancing skin elasticity, preventing or reducing wrinkles, preventing or improving skin aging, promoting hair growth, and restoring miniaturized hair follicles, as well as promoting wound healing.

According to the present invention, the stem cell culture medium not only contains a large amount of exosomes but also provides a significant amount of nano-sized exosomes that penetrate the epidermal layer of the skin due to their unique lipid bilayer structure. These exosomes contain not only anti-inflammatory cytokines but also various growth factors. As a result, they can exert anti-inflammatory effects, promote skin regeneration and anti-aging effects through the proliferation and activation of fibroblasts, increase collagen synthesis, promote hair growth, restore miniaturized hair follicles, and facilitate wound healing.

The present invention enables the mass production of exosomes of approximately 100 nm in size that penetrate the epidermis through the cultivation of umbilical cord blood stem cells. The umbilical cord blood-derived stem cell culture medium produced according to the present invention has high permeability through biomembranes or covering and lining membranes such as the skin, making it desirable for formulations (e.g., pharmaceutical compositions, cosmetics, non-prescription pharmaceutical composition) for coating covering and lining membranes, as well as for therapeutic agents such as anti-inflammatory agents. Moreover, it exhibits an inducing effect on regeneration, demonstrating excellent efficacy when used.

Therefore, stem cell culture medium containing a high concentration of anti-inflammatory cytokine-containing exosomes secreted during the cultivation of umbilical cord blood stem cells or exosomes isolated therefrom according to the present invention can serve as effective components in compositions for topical application to covering and lining membranes such as the skin or compositions for anti-inflammatory and regenerative induction.

The exosomes of the present invention can be produced or used in the form of a culture medium containing them. They can also be produced or used in a state where cells have been removed from the culture medium. Exosome-containing cell-free culture medium poses minimal risks of carcinogenesis and immune rejection and does not pose concerns about occlusion of microvessels when administered systemically. Furthermore, as a non-cellular substance, it allows the development of off-the-shelf products for drug development, thereby reducing manufacturing costs.

### 6. Various Formulations

According to the present invention, the exosome-containing culture medium and/or isolated exosomes secreted by stem cells treated with lipopolysaccharide (LPS) and/or lipoteichoic acid (LTA) during cultivation can be formulated in various ways depending on the intended use.

For example, the exosome-containing culture medium and/or isolated exosomes from the culture medium can be gelled or processed into a powdered form through freezing and/or drying.

They can be used as compositions for coating the skin or other biomembranes (covering and lining membranes), allowing modulation of gelation and solation based on the viscosity difference resulting from the composition ratio of components such as hydrogels and gelatin. Additionally, they can be designed to gel at 4°C in combination with ingredients like poloxamer and solate at body temperature.

Cosmetic composition can be prepared in various forms, including solutions, topical ointments, creams, foams, nourishing lotions, emollient lotions, perfumes, facial masks, emulsions, liquid solutions, makeup bases, essences, soaps, liquid cleansers, bath products, sunscreens, sun oils, suspensions, pastes, gels, lotions, powders, soaps, surfactant-containing cleansers, oils, powder foundations, liquid foundations, wax foundations, patches, and sprays, among others. The choice of formulation is not limited to these examples.

Cosmetic compositions may include one or more cosmetically acceptable vehicles that are commonly used in skincare products. These vehicles may include, but are not limited to, oils, water, surfactants, moisturizers, lower alcohols, thickeners, chelating agents, colorants, preservatives, fragrances, and other suitable ingredients.

The cosmetically acceptable vehicles included in cosmetic compositions can vary depending on the formulation type.

For formulations such as ointments, pastes, creams, or gels, suitable vehicle components may include animal or plant oils, waxes, paraffin, starch, traganth, cellulose derivatives, polyethylene glycol, silicones, bentonite, silica, talc, zinc oxide, or a combination thereof. These components can be used alone or in combination with two or more ingredients.

For formulations in the form of powders or sprays, lactose, talc, silica, aluminum hydroxide, calcium silicate, polyamide powder, or other suitable ingredients may be used as vehicle components. In particular, sprays may also contain propellants such as chlorofluorocarbons, propane/butane, or dimethyl ether, among others. These components can be used alone or in combination with two or more ingredients.

For formulations in the form of solutions or suspensions, the vehicle components may include solvents, solubilizers, or suspending agents. Examples of such components include water, glycerin, ethanol, isopropanol, ethyl carbonate, ethyl acetate, benzyl alcohol, benzyl benzoate, propylene glycol, 1 ,3-butylene glycol, cottonseed oil, peanut oil, corn germ oil, olive oil, pomegranate oil, sesame oil, glyceryl fatty acid esters, polyethylene glycol, or sorbitan fatty acid esters. These components can be used alone or in combination with two or more ingredients.

For formulations in the form of suspensions, the vehicle components may include water, glycerin, ethanol, propylene glycol, ethoxylated isostearyl alcohol, polyoxyethylene sorbitol esters, polyoxyethylene sorbitan ester, or other suspending agents. Additionally, microcrystalline cellulose, aluminum meta-hydroxide, bentonite, agar, or traganth may be used as suspending agents. These components can be used alone or in combination with two or more ingredients.

For formulations in the form of soaps, the vehicle components may include alkali metal salts of fatty acids, fatty acid esters of polyhydric alcohols, hydrolyzed protein fatty acid condensates, isethionates, lanolin derivatives, fatty alcohols, plant oils, glycerol, sugars, or other suitable ingredients. These components can be used alone or in combination with two or more ingredients.

In a cosmetic composition, the exosomes can be included in an amount ranging from 0.0001 to 50% by weight of the total weight of the cosmetic composition. More specifically, they can be included in an amount ranging from 0.0005 to 10% by weight. When the exosomes are included within this range, they exhibit excellent efficacy in improving skin conditions and contribute to the stabilization of the composition.

Generally, non-prescription pharmaceutical composition refer to items used for diagnosing, treating, improving, alleviating, managing, or preventing diseases in humans or animals, excluding those with significant pharmacological effects or items used strictly as medicines. This category includes products used for disease treatment or prevention, as well as those with mild effects on the human body or products that do not directly act on the body.

The non-prescription pharmaceutical composition can be formulated in various forms, such as body cleanser, foam, soap, mask, ointment, cream, lotion, essence, and spray. However, this is not an exhaustive list, and other forms can also be utilized.

In the case of the present invention being a composition for application to covering and lining membranes, such as the skin, for anti-inflammatory and/or regenerative purposes, in addition to including exosomes as active ingredients, it is possible to add a pharmaceutically acceptable carrier.

"Pharmaceutically acceptable" means that the compound, when administered, does not stimulate the organism and does not adversely affect the biological activity and characteristics of the administered compound. It refers to substances that can be commonly used in the pharmaceutical field.

The dosage can be a pharmacologically effective amount for improving skin conditions. The term "pharmacologically effective amount" refers to a quantity sufficient to provide a reasonable benefit-to-risk ratio for medical treatment, and the effective dosage level can be determined based on factors such as the type and severity of the organism, age, sex, type of disease, activity of the drug, sensitivity to the drug, administration time, route of administration, elimination rate, duration of treatment, factors including concurrent use of other drugs known in the medical field. Moreover, the effective amount may vary depending on factors such as the route of administration, the use of excipients, and the possibility of concomitant use with other agents, as recognized by those skilled in the art.

The type of carrier is not specifically limited, and any carrier commonly used in the relevant technical field can be used. Without limitation, examples of carriers include saline solution, sterilized water, Ringer's solution, buffered saline solution, albumin injection solution, lactose, dextrose, sucrose, sorbitol, mannitol, xylitol, erythritol, maltitol, maltodextrin, glycerol, ethanol, among others. These can be used alone or in combination of two or more.

Furthermore, if necessary, other pharmaceutically acceptable additives such as excipients, diluents, antioxidants, buffering agents, or preservatives can be added. Additionally, fillers, thickening agents, humectants, solubilizers, dispersants, surfactants, binders, or lubricants can be optionally added.

In the composition for application to the skin or other covering and lining membranes, the exosomes of the present invention can be used as dermal delivery vehicles.

On the other hand, the transdermal route of administration is commonly used to achieve systemic effects by applying drugs to the skin through transdermal patches. The absorption rate can vary greatly depending on the physical characteristics of the application site skin and the lipophilicity of the drug.

The anti-inflammatory composition and/or the composition for application to the skin or other covering and lining membranes according to the present invention can take the form of exosome-containing transdermal patches. The transdermal patch may include a backing film that protects the product upon skin attachment, a drug reservoir that contains the majority of the drug, a drug-release membrane that controls the drug's release when penetrated, and a contact adhesive layer. Additionally, a release liner that is removed when the product is used as a protective film during packaging can also be included.

The drug-release membrane can be a polymer film that regulates the absorption rate of the drug as it permeates. Furthermore, the contact adhesive layer can be a low-irritation pressure-sensitive adhesive (PSA) designed to adhere to the skin for up to 7 days.

The present invention provides high-concentration exosomes with enhanced anti-inflammatory properties derived from stem cells with induced anti-inflammatory responses, by pre-treating stem cells with lipopolysaccharide (LPS) and/or lipoteichoic acid (LTA) during cultivation to induce a mild inflammatory response and activate the immune response of the stem cells. Furthermore, exosomes derived from stem cells with induced anti-inflammatory responses and high levels of tissue regeneration substances can be used to induce tissue regeneration. The exosomes of the present invention can be utilized as effective components in various formulations, in addition to anti-inflammatory and/or regenerative compositions, such as compositions for topical application to the skin or other covering and lining membranes of a living body, in various dosage forms.

Hereinafter, the present invention will be described in more detail through examples. These examples are for illustrative purposes only, and it will be apparent to those of ordinary skill in the art that the scope of the present invention is not construed as being limited by these examples.

### [Materials]

Lipopolysaccharide (LPS) was purchased from Merck Sigma-Aldrich (cat no. L1329), and its biological source is Escherichia coli (O127:B8). Lipoteichoic acid (LTA) was purchased from Merck Sigma-Aldrich (cat no. L2515), and its biological source is Staphylococcus aureus.

### Example 1: Cell Culture

### 1.1 Umbilical cord blood stem cells

Umbilical cord blood stem cells were cultured in DMEM/F12 medium containing 10% fetal bovine serum (FBS) and fibroblast growth factor 2 (FGF2) at 37°C in a 5% CO₂ incubator.

### 1.2 Human keratinocytes and human fibroblasts

Human keratinocytes were cultured in RPMI-1640 medium containing 10% fetal bovine serum (FBS) at 37°C in a 5% CO₂ incubator. Human fibroblasts were cultured in DMEM medium containing 10% fetal bovine serum (FBS) at 37°C in a 5% CO₂ incubator.

### Example 2: Production of Umbilical Cord Blood Stem Cell Culture Conditioned Media

### 2.1 Cell Culture

Cultured umbilical cord blood stem cells were washed with PBS and then suspended using Trypsin-EDTA. The cell suspension was mixed with twice the volume of culture medium containing Trypsin-EDTA to deactivate the enzyme. After centrifugation, the supernatant was removed, and the cell pellet was resuspended in culture medium. The cell density was measured, and the cells were seeded at a density of 4-10×10³ cells/cm².

### 2.2 Production of Umbilical Cord Blood Stem Cell Culture Supernatant

When the cell confluency reached 60-90%, the culture medium was replaced with serum-free media after washing the culture dish with PBS. The cells were cultured for 2-7 days in serum-free media. After 2-7 days, the supernatant excluding the cells was collected, and centrifugation at 1000xg for 3 minutes was performed to remove cells and debris. The culture supernatant was then filtered through a 0.2 µm filter.

### 2.3 Pre-treatment of Umbilical Cord Blood Stem Cells with LPS and LTA and Production of Culture Conditioned Media from the pre-treated Umbilical Cord Blood Stem Cell

After seeding the umbilical cord blood stem cells, pre-treatment with LPS (Lipopolysaccharide) at a concentration of 10-1000 ng/ml and/or LTA (Lipoteichoic Acid) at a concentration of 10-1000 ng/ml was performed between 12-48 hours, based on the replacement of the culture medium with serum-free media. After pre-treatment, the culture dish was washed with PBS and the medium was replaced with serum-free media. The cells were then cultured for 2-7 days. After 2-7 days, the supernatant excluding the cells was collected, and centrifugation at 1000xg for 3 minutes was performed to remove cells and debris. The culture supernatant was then filtered through a 0.2 µm filter.

### 2.4 Analysis of Inflammation Inhibitory Factor Expression in Pre-treated Umbilical Cord Blood Stem Cells and Culture Supernatant

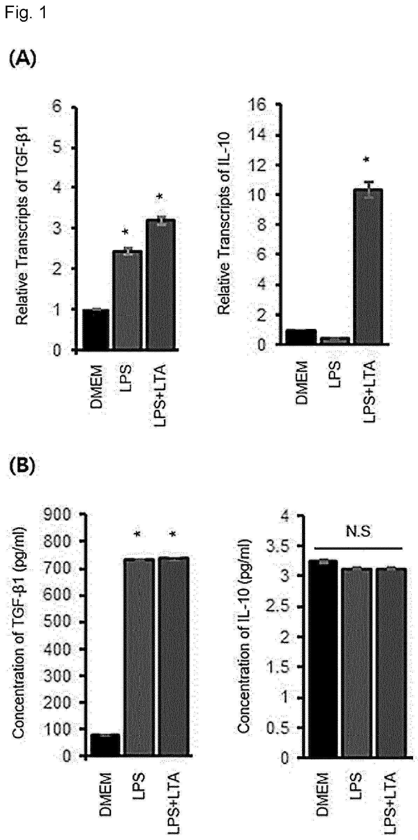

The expression of anti-inflammatory factors in pre-treated umbilical cord blood stem cells and culture supernatant was analyzed. As shown in Fig. 1, (A) the mRNA expression of anti-inflammatory factors TGF-β1 and IL-10 was increased in umbilical cord blood stem cells pre-treated with LPS and LTA(Example 2.3) compared to the control group (Example 2.2). (B) The concentration of TGF-β1 in the culture supernatant produced after pre-treatment with LPS and LTA was increased compared to the control group, while no significant difference was observed in IL-10 concentration among the experimental groups.

### Example 3: Isolation of Umbilical Cord Blood Stem Cell Exosomes

Exosomes were extracted from the filtered culture supernatant obtained from each condition in Example 2 using the Capturem^{™} Exosome Isolation Kit (Takara Bio).

### 3.1 Analysis of Concentration and Size of Umbilical Cord Blood Stem Cell Exosomes Produced with LPS and LTA Pre-treatment

The size distribution and concentration of exosomes extracted from the culture media of umbilical cord blood stem cells cultured under each condition were measured using the Nanosight_NS300 system (Malvern).

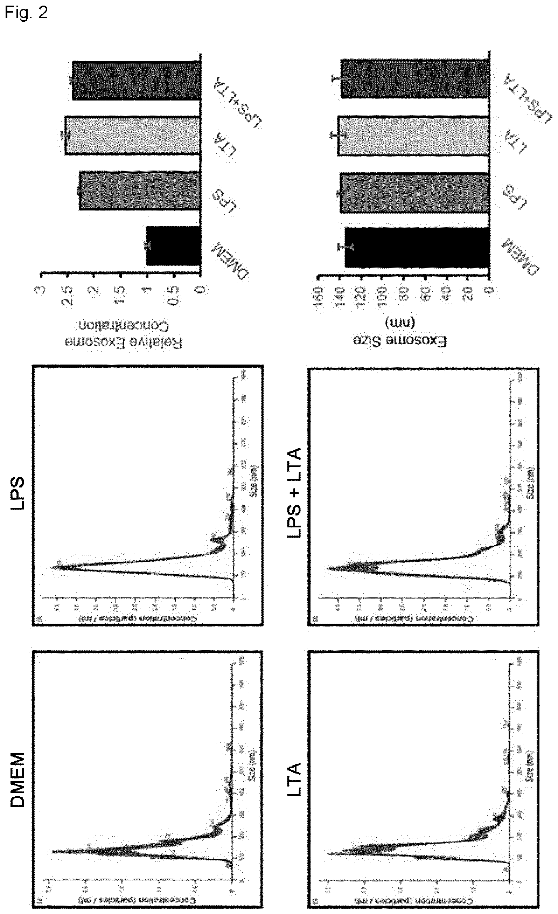

As shown in Fig. 2, the concentration of exosomes isolated from umbilical cord blood stem cells pre-treated with LPS and/or LTA was higher compared to the control group, indicating an increase in exosome concentration. However, the size of the exosomes was similar to that of the control group.

### 3.2 Analysis of Inflammatory Factor Content in Umbilical Cord Blood Stem Cell Exosomes Produced with LPS and LTA Pre-treatment

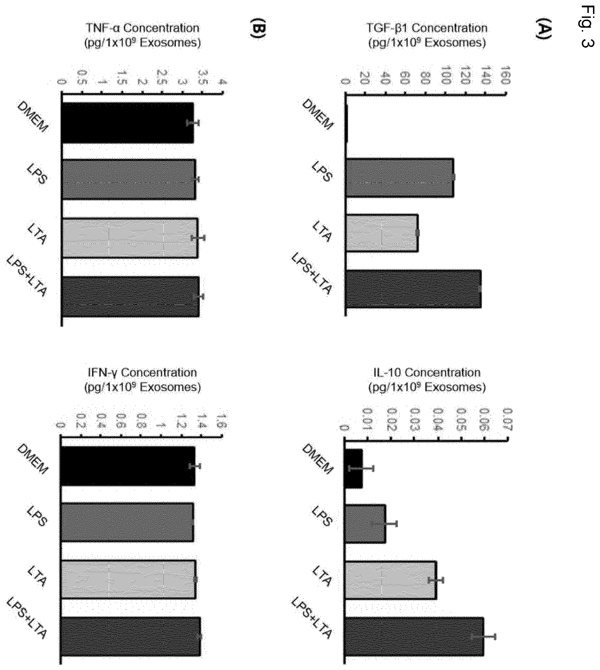

The content of inflammatory factors present in umbilical cord blood stem cell exosomes under each condition was analyzed using ELISA. As shown in Fig. 3, the analysis of exosomes from each condition revealed that (A) the content of anti-inflammatory factors, TGF-β1 and IL-10, was higher in exosomes derived from umbilical cord blood stem cells pre-treated with LPS and LTA compared to the untreated control group (DMEM). However, (B) the content of pro-inflammatory factors, TNF-α and IFN-γ, showed no significant difference compared to the control group.

### Example 4. Analysis of Anti-inflammatory Efficacy of Exosomes in Human Keratinocytes

### 4.1 Cell Viability Analysis with Exosome Treatment in Inflammation-Induced Human Keratinocytes

Human Keratinocytes were seeded in a 12-well plate and cultured in RPMI-1640 medium containing 10% fetal bovine serum until reaching 80% cell confluence. The medium was then replaced with serum-free RPMI-1640 basal media containing TNF-α (10 ng/ml) and IFN-γ (10 ng/ml) to induce inflammation. After 6 hours of media replacement, exosomes extracted from the respective culture supernatant mentioned in Example 2 were added to the inflammation-induced human keratinocytes at concentrations ranging from 1 to 10×10⁸ exosomes. The cells were cultured for 24 hours, and cell viability was determined using the CCK-8 assay (A).

Additionally, human keratinocytes were pre-treated with exosomes from each condition mentioned in Example 2 for 6 hours, followed by treatment with inflammatory agents TNF-α and IFN-γ at a concentration of 10 ng/ml. After 24 hours, cell viability was analyzed (B).

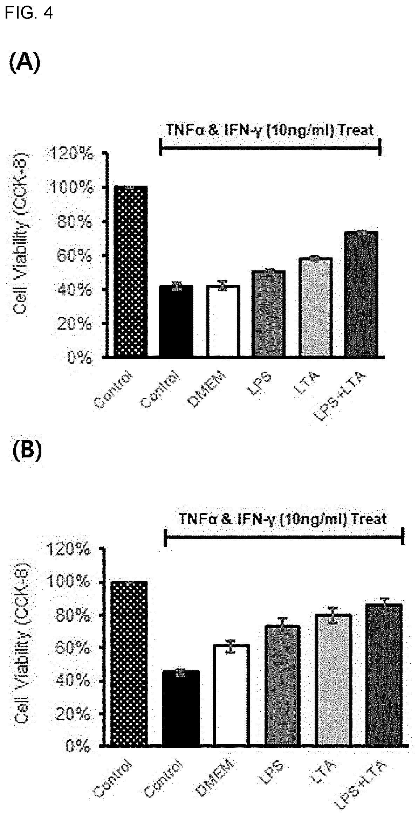

As shown in Fig. 4(A), when the human keratinocytes were pre-treated with inflammatory agents TNF-α and IFN-γ at a concentration of 10 ng/ml for 6 hours and then treated with exosomes from each condition mentioned in Example 2, a significant decrease in cell viability of the inflammation-induced control group (second control within the graph) was observed compared to the non-treated control (first control within the graph). However, the experimental groups treated with exosomes from each condition in Example 2.3 showed recovery of cell viability.

In Fig. 4(B), when human keratinocytes were pre-treated with exosomes from each condition mentioned in Example 2 and then treated with TNF-α and IFN-γ at a concentration of 10 ng/ml for 6 hours, the cell viability was assessed after 24 hours and as a result, a significant decrease in cell viability of the inflammation-induced control group (second control within the graph) was observed compared to the non-treated control (first control within the graph). However, the experimental groups treated with exosomes from each condition in Example 2.3 showed recovery of cell viability.

Furthermore, as shown in Fig. 4(A) and (B), the experimental groups (LPS, LTA, LPS+LTA) treated with exosomes produced by pre-treating with LPS and LTA did not show a decrease in cell viability, indicating that LPS and LTA may not be present in the exosomes.

### 4.2 Anti-inflammatory Efficacy Confirmation Test

Human keratinocytes were seeded in a 12-well plate and cultured in RPMI-1640 medium containing 10% fetal bovine serum until reaching 80% cell confluency. Then, the medium was replaced with serum-free RPMI-1640 basal media containing TNF-α (10ng/ml) and IFN-γ (10ng/ml) to induce inflammation. After 6 hours of medium replacement, exosomes extracted from each condition in Example 2 were added to the inflamed human keratinocytes at concentrations ranging from 2×10⁸ to 10×10⁸ particles and incubated for 24 hours. The culture medium and cells were collected thereafter.

### 4.2.1 Analysis of Inflammatory Mediator Expression after Exosome Treatment in Inflamed Human Keratinocytes

Total RNA was extracted from the human keratinocytes treated with exosomes from Example 2 after the induction of inflammation. The mRNA expression levels of pro-inflammatory cytokines, TNF-α, and IL-6, were analyzed. The culture medium of the human keratinocytes treated with exosomes from Example 2 after the induction of inflammation was also analyzed for protein expression levels of TNF-α and IL-6 using ELISA.

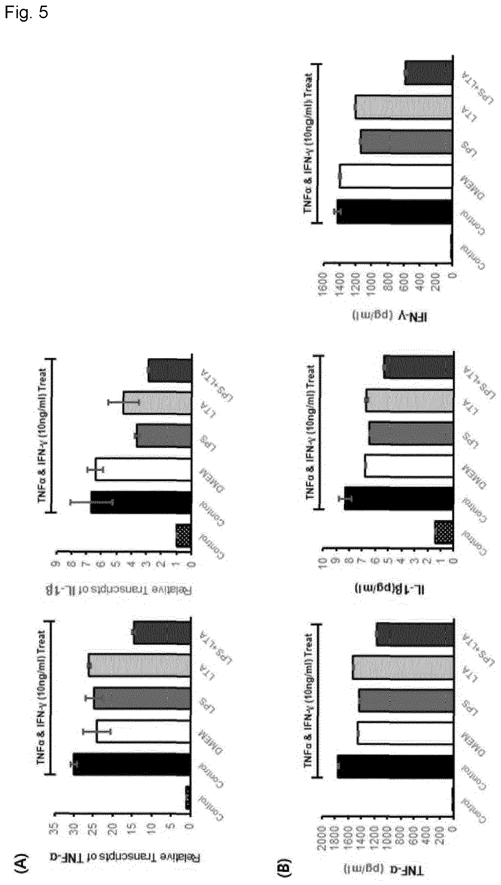

The results of analyzing the expression of inflammatory mediators in inflamed human keratinocytes treated with exosomes from each condition are shown in Fig. 5(A). Compared to the control group without treatment (1st control in the graph), the mRNA expression levels of inflammatory mediators significantly increased in the inflamed control group (2nd control in the graph). However, the experimental groups treated with exosomes from each condition mentioned in Example 2.3 (LPS, LTA, LPS+LTA) showed varying degrees of reduction in the expression levels of inflammatory mediators.

Additionally, Fig. 5(B) demonstrates that the protein expression levels of inflammatory mediators significantly increased in the inflamed control group (2nd control in the graph) compared to the control group without treatment (1st control in the graph). However, the experimental groups treated with exosomes from each condition mentioned in Example 2.3 (LPS, LTA, LPS+LTA) showed varying degrees of reduction in the expression levels of inflammatory mediators.

Particularly, the group treated with exosomes pre-treated with both LPS and LTA exhibited a further decrease in the expression levels of inflammatory mediators compared to the control group without treatment, indicating an enhanced anti-inflammatory effect of exosomes through the pre-treatment with LPS and LTA.

### 4.2.2 Analysis of Anti-inflammatory Mediator Expression after Exosome Treatment in Inflamed Human Keratinocytes

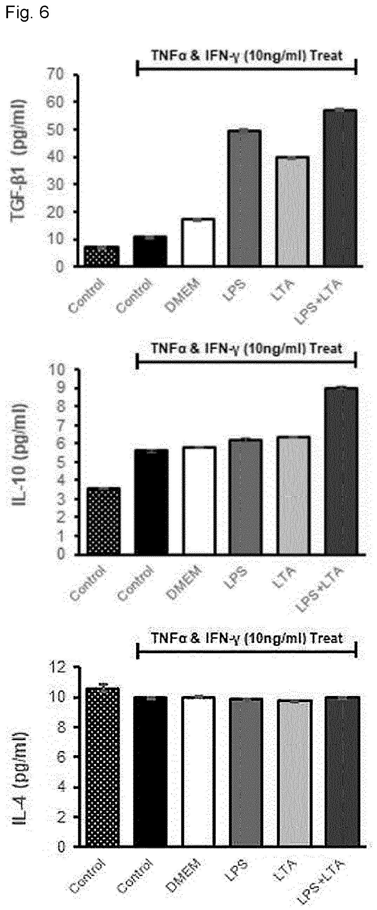

The expression of anti-inflammatory mediators in inflamed human keratinocytes treated with exosomes from each condition in Example 2.3 was analyzed. Fig. 6 illustrates that the expression of anti-inflammatory mediators was modulated in various patterns in the experimental groups treated with exosomes from each condition in Example 2.3 (LPS, LTA, LPS+LTA).

Particularly, the group treated with exosomes pre-treated with both LPS and LTA showed an increased expression of anti-inflammatory mediators, TGF-β1 and IL-10, compared to the untreated control group, indicating an enhanced anti-inflammatory efficacy of exosomes through the pre-treatment with LPS and LTA. However, there was no significant increase in the expression of IL-4, an inflammatory inducer.

### Example 5: Analysis of Regenerative Induction Efficacy after Exosome Treatment

Using exosomes isolated after simultaneous treatment of LPS and LTA under the conditions of Example 2, the cell proliferation ability and cell migration ability, which are indicative of tissue regeneration, were analyzed.

### 5.1 Analysis of Cell Proliferation Ability in Human Keratinocytes and Human Fibroblasts after Exosome Treatment

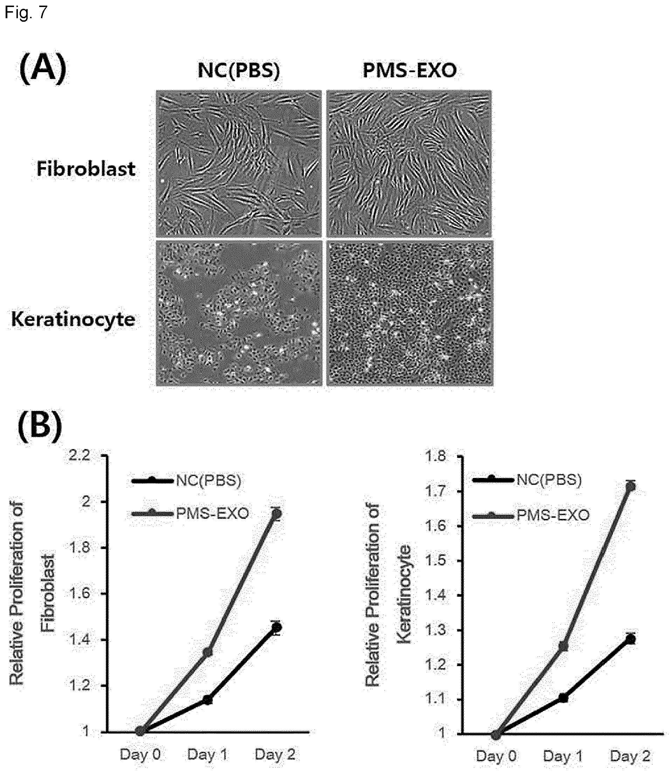

Human keratinocytes and human fibroblasts were coated in culture dishes containing RPMI-1640 and DMEM medium, respectively, supplemented with 10% fetal bovine serum. The cells were allowed to attach for one day, and the following day they were treated with exosomes. Cell viability was measured using CCK-8 at 24-hour intervals. The experimental groups treated with exosomes showed significantly faster cell proliferation rates compared to the control groups in both cell types (Fig. 7).

### 5.2 Analysis of Cell Migration Ability in Human Keratinocytes and Human Fibroblasts after Exosome Treatment

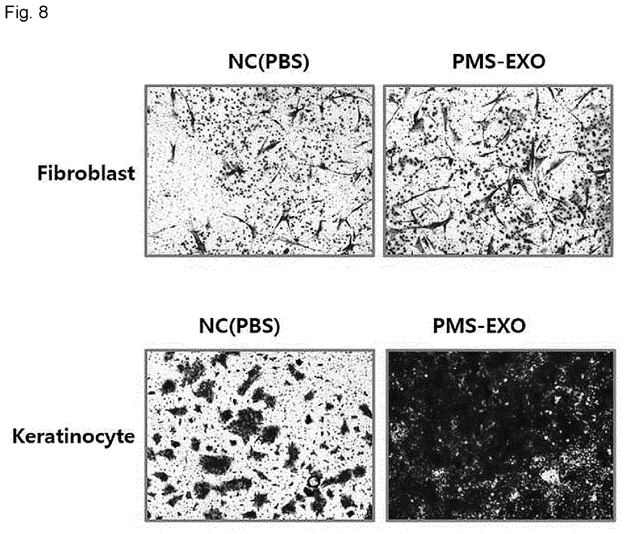

Human keratinocytes and human fibroblasts were seeded on Transwell inserts with porous membranes until reaching 100% cell confluency, and attachment was induced. Then, exosomes were added, and the cells were cultured for 48 hours, allowing them to migrate through the pores. After a certain period, the opposite side of the membrane was stained with crystal violet to visualize the cells that had migrated through the holes. The results showed that the experimental groups treated with exosomes exhibited significantly higher staining intensity compared to the control groups, indicating enhanced cell migration facilitated by exosomes (Fig. 8).

## Claims

1. A method of producing exosomes comprising:
(a) a step of culturing umbilical cord blood stem cells in a serum-free medium supplemented with one or more selected from a group consisting of lipopolysaccharide (LPS) and lipoteichoic acid (LTA) to increase the concentration of exosomes secreted during the culture of umbilical cord blood stem cells compared to untreated umbilical cord blood stem cells; and
(b) optionally, a step of separating umbilical cord blood stem cells, their culture conditioned medium, and/or their exosomes obtained from step (a).

2. The method of producing exosomes according to claim 1, wherein the exosomes have enhanced anti-inflammatory and/or regenerative functions compared to exosomes derived from the untreated umbilical cord blood stem cells.

3. A composition for topical application to the skin or other covering and lining membranes of the body, comprising exosomes obtained by the production method described in claim 1 or claim 2, or a culture conditioned medium containing said exosomes as an active ingredient.

4. The composition for topical application according to claim 3, which is a cosmetic composition, a non-prescription pharmaceutical composition, or a pharmaceutical composition.

5. The composition for topical application according to claim 3, wherein the exosomes secreted from umbilical cord blood stem cells are used as transdermal carriers.

6. The composition for topical application according to claim 3, wherein the composition is formulated as a gelatinized dosage form, which is mixed with a polymer and undergoes sol-gel transition at body temperature.

7. A method of producing an anti-inflammatory and/or regenerative composition comprises the following steps:
(i) treating stem cells during cultivation with one or more selected from the group consisting of lipopolysaccharide (LPS) and lipoteichoic acid (LTA); and
(ii) optionally, separating stem cells, their culture conditioned medium, and/or their exosomes obtained from step (i).

8. The method of producing an anti-inflammatory and/or regenerative composition according to claim 7, wherein step (i) involves treating stem cells during cultivation with both lipopolysaccharide (LPS) and lipoteichoic acid (LTA).

9. The method of producing an anti-inflammatory and/or regenerative composition according to claim 7, wherein lipopolysaccharide (LPS) is a component of the cell wall in Gram-negative bacteria and/or lipoteichoic acid (LTA) is a component of the cell wall in Gram-positive bacteria.

10. The method of producing an anti-inflammatory and/or regenerative composition according to claim 7, wherein by treating with lipopolysaccharide (LPS) and/or lipoteichoic acid (LTA) in step (i), the mRNA expression and/or secretion of anti-inflammatory cytokines are increased and/or the secreted exosomes exhibit a higher content of anti-inflammatory cytokines, compared to untreated stem cells during stem cell cultivation.

11. The method of producing an anti-inflammatory and/or regenerative composition according to claim 7, wherein the activation of the immune response in stem cells is achieved by treating them with lipopolysaccharide (LPS) and/or lipoteichoic acid (LTA) in step (i).

12. The method of producing an anti-inflammatory and/or regenerative composition according to claim 7, wherein in step (i), lipopolysaccharide (LPS) and/or lipoteichoic acid (LTA) induce the inflammatory response in stem cells.

13. The method of producing an anti-inflammatory and/or regenerative composition according to claim 7, wherein the resulting exosomes from step (i) do not contain LPS and/or LTA.

14. The method of producing an anti-inflammatory and/or regenerative composition according to claim 7, wherein when the resulting product of step (i) is applied to cells, it reduces the expression of pro-inflammatory factors and/or increases the expression of anti-inflammatory factors.

15. The method of producing an anti-inflammatory and/or regenerative composition according to claim 7, wherein the anti-inflammatory composition is the exosomes secreted by stem cells, as the result of step (i) or a culture medium containing these exosomes.

16. The method of producing an anti-inflammatory and/or regenerative composition according to claim 7, wherein in step (i), the production of exosomes secreted by stem cells is increased by lipopolysaccharide (LPS) and/or lipoteichoic acid (LTA).

17. The method of producing an anti-inflammatory and/or regenerative composition according any one of claims 7 to 16, wherein the stem cells are umbilical cord blood stem cells.

18. An anti-inflammatory and/or regenerative composition obtained by the method of any one of claims 7 to 16.
